(19) Europäisches Patentamt
European Patent Office
Office européen des brevets

(11) Veröffentlichungsnummer: **0 188 194**
**B1**

(12) **EUROPÄISCHE PATENTSCHRIFT**

(45) Veröffentlichungstag der Patentschrift: **04.07.90**

(21) Anmeldenummer: **86100052.9**

(22) Anmeldetag: **03.01.86**

(51) Int. Cl.⁵: **C 07 C 229/30,**
C 07 C 69/738, C 07 C 49/84,
C 07 C 49/825, C 07 C 227/04,
C 07 C 67/31, C 07 C 67/00,
C 07 C 45/68, C 07 D 215/48,
C 07 D 215/56

# (54) Aminoacrylsäure-Derivate.

(30) Priorität: **16.01.85 DE 3501247**

(43) Veröffentlichungstag der Anmeldung:
**23.07.86 Patentblatt 86/30**

(45) Bekanntmachung des Hinweises auf die Patenterteilung:
**04.07.90 Patentblatt 90/27**

(84) Benannte Vertragsstaaten:
**AT BE CH DE FR GB IT LI NL SE**

(56) Entgegenhaltungen:
**EP-A-0 078 362**
**EP-A-0 168 733**

(73) Patentinhaber: **BAYER AG**
**D-5090 Leverkusen 1 Bayerwerk (DE)**

(72) Erfinder: **Maurer, Fritz, Dr.**
**Roeberstrasse 8**
**D-5600 Wuppertal 1 (DE)**
Erfinder: **Grohe, Klaus, Dr.**
**Am Wasserturm 10**
**D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 188 194 B1

**Beschreibung**

Die vorliegende Erfindung betrifft neue Aminoacrylsäure-Derivate, ein Verfahren zu ihrer Herstellung, sowie ihre Verwendung als Zwischenprodukt zur Herstellung von Oxochinolincarbonsäure-Derivaten.

Es ist bereits bekannt, daß man Oxochinolincarbonsäure-Derivate erhalten kann, wenn man Aminoacrylsäure-Derivate als Zwischenprodukte einsetzt. Die Herstellung dieser Aminoacrylsäure-Derivate ist jedoch aufwendig und erfolgt in mehreren Stufen (vgl. z.B. EP—OS 78 362).

Die vorliegende Erfindung betrifft:

1. Neue Aminoacrylsäure-Derivate der Formel (I)

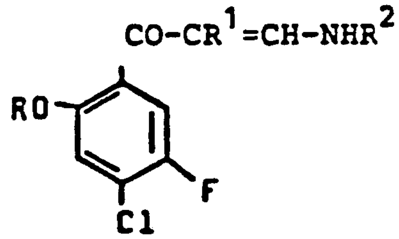

(I)

in welcher

R für Alkyl steht,

$R^1$ für Alkoxycarbonyl steht und

$R^2$ für Alkyl, Cycloalkyl, Amino, Alkylamino oder Di-alkylamino steht.

2. Ein Verfahren zur Herstellung der neuen Aminoacrylsäure-Derivate der Formel (I)

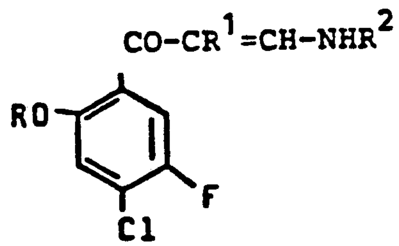

(I)

in welcher

R für Alkyl steht,

$R^1$ für Alkoxycarbonyl steht und

$R^2$ für Alkyl, Cycloalkyl, Amino, Alkylamino oder Di-alkylamino steht.

das dadurch gekennzeichnet ist, daß man Acrylsäure-Derivate der Formel (II)

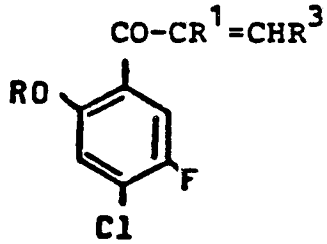

(II)

in welcher

R und $R^1$ die bei 1 (oben) angegebene Bedeutung haben und

$R^3$ für Alkoxy steht

mit Aminen der Formel (III)

$$R^2NH_2 \qquad (III)$$

in welcher

$R^2$ die bei 1 (oben) angegebene Bedeutung hat,

gegebenenfalls in Gegenwart von inerten Verdünnungsmitteln umsetzt.

3. Acrylsäure-Derivate der Formel (II)

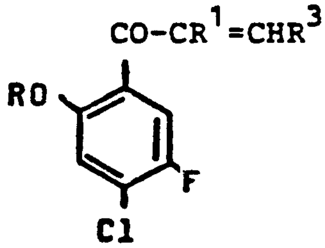

(II)

in welcher
R, $R^1$ und $R^3$ die bei 2 (oben) angegebenen Bedeutungen haben.

4. Ein Verfahren zur Herstellung der neuen Acrylsäure-Derivate der Formel (II)

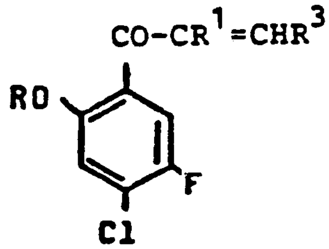

(II)

in welcher
R, $R^1$ und $R^3$ die bei 2 (oben) angegebenen Bedeutungen haben,
das dadurch gekennzeichnet ist, daß man 2-Alkoxy-4-chlor-5-fluor-benzoylessigsäureester der Formel (IV)

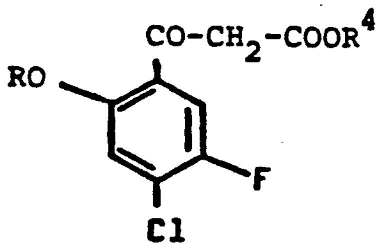

(IV)

in welcher
R die oben angegebene Bedeutung hat und
$R^4$ für Alkyl steht,
mit Orthomeisensäureestern der Formel (V)

$$HC(R^3)_3 \qquad (V)$$

in welcher
$R^3$ die oben angegebene Bedeutung hat,
in Gegenwart von Acetanhydrid und gegegenenfalls in Gegenwart von Verdünnungsmitteln umsetzt.

5. 2-Alkoxy-4-chlor-5-fluor-benzoylessigsäureester der Formel (IV)

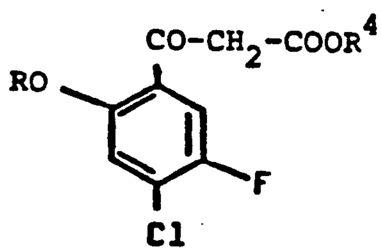

(IV)

in welcher
R und $R^4$ die bei 4 (oben) angegebenen Bedeutungen haben.

6. Ein Verfahren zur Herstellung der neuen 2-Alkoxy-4-chlor-5-fluor-benzoylessigsäureester der Formel (IV),

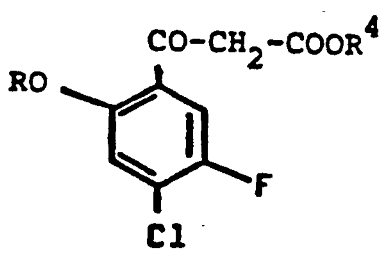

(IV)

in welcher
R und $R^4$ die bei 4 (oben) angegebenen Bedeutungen haben,
das dadurch gekennzeichnet ist, daß man Acetophenone der Formel (VI)

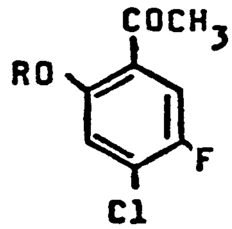

(VI)

in welcher
R die bei 5 (oben) angegebene Bedeutung hat, mit Kohlensäuredialkylestern der Formel (VII)

$R^4—O—CO—OR^4$ (VII)

in welcher
$R^4$ die bei 5 (oben) angegebene Bedeutung hat,
in Gegenwart von starken Basen umsetzt.

7. Acetophenone der Formel (VI)

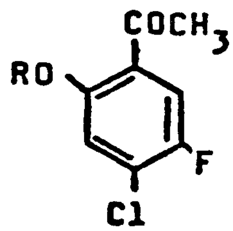

(VI)

in welcher
R die bei 5 (oben) angegebene Bedeutung hat.

8. Ein Verfahren zur Herstellung der Acetophenone der Formel (IV),

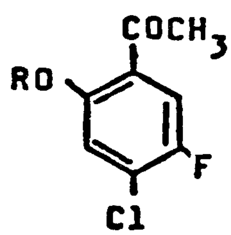

(VI)

in welcher
R die bei 5 (oben) angegebene Bedeutung hat,
das dadurch gekennzeichnet ist, daß man 4-Chlor-5-fluor-2-hydroxacetophenon der Formel (VIII)

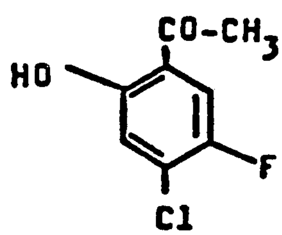

(VIII)

α) mit Allylhalogeniden der Formel (IX)

R—Hal (IX)

in welcher
R die oben angegebene Bedeutung hat und
Hal für Halogen steht, oder

β) mit Dialkylsulfaten der Formel (X)

$RO—SO_2—OR$ (X)

in welcher
R die oben angegebene Bedeutung hat,
in Gegenwart von Säureakzeptoren und in Gegenwart von Verdünnungsmitteln umsetzt.

9. 4-Chlor-5-fluor-2-hydroxyacetophenon der Formel (VIII)

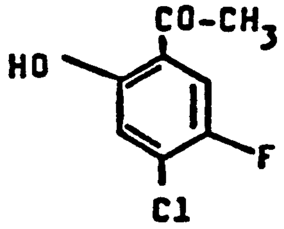

(VIII)

10. Verfahren zur Herstellung von 4-Chlor-5-fluor-2-hydroxyacetophenon der Formel (VIII), dadurch gekennzeichnet, daß man 3-Chlor-4-fluorphenol der Formel (XI)

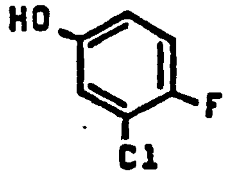

(XI)

mit Acetylierungsmitteln der Formel (XII)

$$CH_3\overset{\overset{O}{\|}}{C}—W \qquad \text{(XII)}$$

in welcher

W für Halogen oder den Rest $CH_3$—COO— steht,

in Gegenwart von Acylierungskatalysatoren und gegebenenfalls in Gegenwart von Verdünnungsmitteln umsetzt.

11. Verwendung von Verbindungen der Formel (I) gemäß 1 (oben), zur Herstellung von Oxochinolincarbonsäure-Derivaten, dadurch gekennzeichnet, daß man die Verbindungen der Formel (I) cyclisiert und anschließend nach bekannten Methoden zu Oxochinolincarbonsäure-Derivaten umsetzt (vgl. z.B. EP—OS 78 362).

Überraschenderweise können mit Hilfe der erfindungsgemäßen Verbindungen der Formel (I) Oxochinolincarbonsäure-Derivate auf einfachere Weise und somit kostengünstiger hergestellt werden, als aus dem Stand der Technik bekannt ist (vgl. EP—OS 78 362). Nach dem Stand der Technik werden die substituierten Benzoylessigsäureethylester hergestellt, indem man z.B. Trihalogenmethylbenzol-Derivate verseift, die Säure mit Thionylchlorid in das entsprechende Benzoylchlorid überführt und danach mit Malonsäurediethylester in Gegenwart von Magnesiumalkoholat zum Benzoylmalonester-Derivat umsetzt. Anschließend wird durch partielle Verseifung und Decarboxylierung im wäßrigen Medium mit katalytischen Mengen p-Toluolsulfonsäure der Benzoylessigsäureethylester gebildet. Die weitere Umsetzung der Benzoylessigsäureethylester erfolgt zunächst analog zu dem in EP—OS 78 362 angegebenen Verfahren durch Umsetzung mit O-Ameisensäureester und anschließende Umsetzung mit Aminen. Danach erfolgt ein Ringschluß in Gegenwart von Säureakzeptoren und unter Abspaltung von Alkohol. Diese Verbindungen können anschließend nach bekannten Methoden zu den entsprechenden Oxochinolincarbonsäure-Derivaten weiterreagieren (vgl. z.B. EP—OS 78 362).

Die Erfindung betrifft vorzugsweise Verbindungen der Formel (I),

in welcher

R für $C_1$—$C_4$-Alkyl steht,

$R^1$ für $C_1$—$C_4$-Alkoxycarbonyl steht und

$R^2$ für $C_1$—$C_4$-Alkyl, $C_3$—$C_6$-Cycloalkyl, Amino $C_1$—$C_4$-Alkylamino oder Di-($C_1$—$C_4$)-Alkylamino steht.

Besonders bevorzugt sind die Verbindungen der Formel (I)

in welcher

R für Methyl, Ethyl oder n-Propyl steht,

$R^1$ für Methoxycarbonyl, Ethoxycarbonyl oder n-Propoxycarbonyl steht und

$R^2$ für Methyl, Ethyl, Cyclopropyl, Amino, ($C_1$—$C_2$)-Alkylamino oder Di-($C_1$—$C_2$)-Alkylamino steht.

Verwendet man beispielsweise für das erfindungsgemäße Verfahren (2) zur Herstellung der erfindungsgemäßen Verbindungen der Formel (I) 3-Ethoxy-2-(4-chlorfluor-2-ethoxy-benzoyl)-acrylsäureethylester und Cyclopropylamin als Ausgangsstoffe, so kann die Reaktion durch das folgende Formelschema wiedergegeben werden:

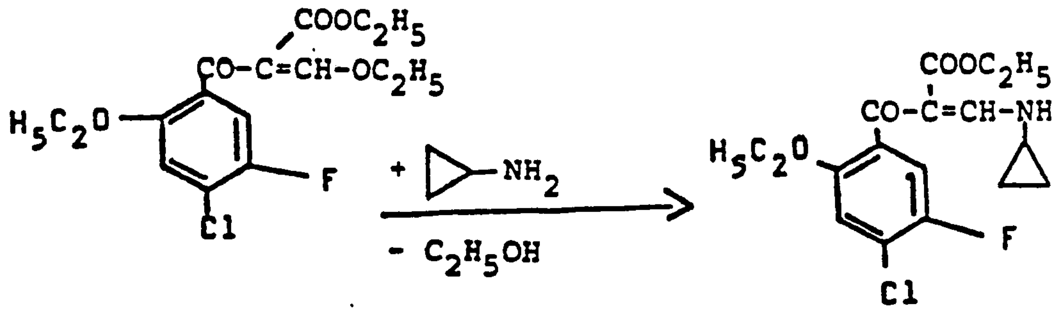


Die als Ausgangsstoffe für das erfindungsgemäße Verfahren (2) zu verwendenden Acrylsäure-Derivate sind durch die Formel (II) allgemein definiert. In dieser Formel stehen R und $R^1$ bevorzugt für diejenigen Reste, welche oben für Formel (I) angegeben sind. $R^3$ steht in dieser Formel für Alkoxy. Bevorzugt steht $R^3$ für $C_1$—$C_4$-Alkoxy.

Die Verbindungen der Formel (II) sind neu. Sie lassen sich gemäß 4 (oben) herstellen (siehe weiter unten).

Als Beispiele für die Verbindungen der Formel (II) seien genannt:

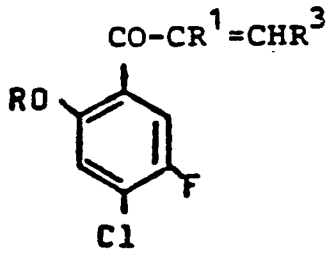

(II)

Tabelle 1

| R | $R^1$ | $R^3$ | R | $R^1$ | $R^3$ |
|---|---|---|---|---|---|
| $CH_3$ | $-COOCH_3$ | $OCH_3$ | $CH_3$ | $-COOCH_3$ | $OC_2H_5$ |
| $C_2H_5$ | $-COOCH_3$ | $OCH_3$ | $C_2H_5$ | $-COOCH_3$ | $OC_2H_5$ |
| $n-C_3H_7$ | $-COOCH_3$ | $OCH_3$ | $n-C_3H_7$ | $-COOCH_3$ | $OC_2H_5$ |
| $i-C_3H_7$ | $-COOCH_3$ | $OCH_3$ | $i-C_3H_7$ | $-COOCH_3$ | $OC_2H_5$ |
| $n-C_3H_9$ | $-COOCH_3$ | $OCH_3$ | $n-C_4H_9$ | $-COOCH_3$ | " |
| $i-C_4H_9$ | $-COOCH_3$ | $OCH_3$ | $i-C_4H_9$ | $-COOCH_3$ | " |
| $sec.-C_4H_9$ | $-COOCH_3$ | $OCH_3$ | $sec.-C_4H_9$ | $-COOCH_3$ | " |
| $tert.-C_4H_9$ | $-COOCH_3$ | $OCH_3$ | $tert.-C_4H_9$ | $-COOCH_3$ | " |
| $CH_3$ | $-COOC_2H_5$ | $OCH_3$ | $CH_3$ | $-COOC_2H_5$ | " |
| $C_2H_5$ | " | " | $C_2H_5$ | " | " |
| $n-C_3H_7$ | " | " | $n-C_3H_7$ | " | " |
| $i-C_3H_7$ | " | " | $i-C_3H_7$ | " | " |
| $n-C_4H_9$ | " | " | $n-C_4H_9$ | " | " |
| $i-C_4H_9$ | " | " | $i-C_4H_9$ | " | " |
| $sec.-C_4H_9$ | " | " | $sec.-C_4H_9$ | " | " |
| $tert.-C_4H_9$ | " | " | $tert.-C_4H_9$ | " | " |

Tabelle 1 (Fortsetzung)

| R | $R^1$ | $R^3$ | R | $R^1$ | $R^3$ |
|---|---|---|---|---|---|
| $CH_3$ | $-COOC_3H_7$-n | $OCH_3$ | $CH_3$ | $-COOC_3H_7$-n | $OC_2H_5$ |
| $C_2H_5$ | " | " | $C_2H_5$ | " | " |
| n-$C_3H_7$ | " | " | n-$C_3H_7$ | " | " |
| i-$C_3H_7$ | " | " | i-$C_3H_7$ | " | " |
| n-$C_4H_9$ | " | " | n-$C_4H_9$ | " | " |
| i-$C_4H_9$ | " | " | i-$C_4H_9$ | " | " |
| sec.-$C_4H_9$ | " | " | sec.-$C_4H_9$ | " | " |
| tert.-$C_4H_9$ | " | " | tert.-$C_4H_9$ | " | " |
| $CH_3$ | $-COOC_3H_7$-i | $OCH_3$ | $CH_3$ | $-COOC_3H_7$-i | " |
| $C_2H_5$ | " | " | $C_2H_5$ | " | " |
| n-$C_3H_7$ | " | " | n-$C_3H_7$ | " | " |
| i-$C_3H_7$ | " | " | i-$C_3H_7$ | " | " |
| n-$C_4H_9$ | " | " | n-$C_4H_9$ | " | " |
| i-$C_4H_9$ | $-COOC_3H_7$-i | $OCH_3$ | i-$C_4H_9$ | $-COOC_3H_7$-i | $OC_2H_5$ |
| sec.-$C_4H_9$ | " | " | sec.-$C_4H_9$ | " | " |
| tert.-$C_4H_9$ | " | " | tert.-$C_4H_9$ | " | " |
| $CH_3$ | $-COOC_4H_9$-n | " | $CH_3$ | $-COOC_4H_9$-n | " |
| $C_2H_5$ | " | " | $C_2H_5$ | " | " |
| n-$C_3H_7$ | " | " | n-$C_3H_7$ | " | " |
| i-$C_3H_7$ | " | " | i-$C_3H_7$ | " | " |
| n-$C_4H_9$ | " | " | n-$C_4H_9$ | " | " |
| i-$C_4H_9$ | " | " | i-$C_4H_9$ | " | " |
| sec.-$C_4H_9$ | " | " | sec.-$C_4H_9$ | " | " |
| tert.-$C_4H_9$ | " | " | tert.-$C_4H_9$ | " | " |
| $CH_3$ | $-COOCH_3$ | $OC_3H_7$-n | $CH_3$ | $-COOC_3H_7$-n | $OC_3H_7$-n |
| | | | $C_2H_5$ | | |
| $C_2H_5$ | " | " | n-$C_3H_7$ | | " |
| | | | i-$C_3H_7$ | | " |
| n-$C_3H_7$ | " | " | n-$C_4H_9$ | | " |
| i-$C_3H_7$ | " | " | i-$C_4H_9$ | | " |
| | | " | sec.-$C_4H_9$ | | " |
| n-$C_4H_9$ | " | " | tert.-$C_4H_9$ | | " |
| | | " | $CH_3$ | $-COOC_3H_7$-i | " |

Tabelle 1 (Fortsetzung)

| R | $R^1$ | $R^3$ |
|---|---|---|
| i-$C_4H_9$ | $-COOCH_3$ | $OC_3H_7$-n |
| | | " |
| sec.-$C_4H_9$ | " | " |
| | | " |
| tert.-$C_4H_9$ | " | " |
| | " | " |
| $CH_3$ | $-COOC_2H_5$ | $OC_3H_7$-n |
| $C_2H_5$ | " | " |
| n-$C_4H_7$ | " | " |
| i-$C_3H_7$ | " | " |
| n-$C_4H_9$ | " | " |
| i-$C_4H_9$ | " | " |
| sec.-$C_4H_9$ | " | " |
| tert.-$C_4H_9$ | " | " |
| $C_2H_5$ | $-COOC_3H_7$-n | $OC_3H_7$-n |
| n-$C_3H_7$ | " | " |
| i-$C_3H_7$ | " | " |
| n-$C_4H_9$ | " | " |
| i-$C_4H_9$ | " | " |
| sec.-$C_4H_9$ | " | " |
| tert.-$C_4H_9$ | $-COOC_3H_7$-i | $OC_3H_7$-n |
| $CH_3$ | $-COOC_4H_9$-n | " |
| $C_2H_5$ | " | " |
| n-$C_3H_7$ | " | " |
| i-$C_3H_7$ | " | " |
| n-$C_4H_9$ | " | " |
| i-$C_4H_9$ | " | " |
| sec.-$C_4H_9$ | " | " |
| tert.-$C_4H_9$ | | |

Die für das erfindungsgemäße Verfahren (2) außerdem als Ausgangsstoffe zu verwendenden Amine sind durch die Formel (III) algemein definiert. In dieser Formel steht $R^2$ bevorzugt für diejenigen Reste, welche oben bei der Formel (I) angegeben sind.

Die Verbindungen der Formel (III) sind allgemein bekannte Verbindungen der organischen Chemie.

Als Beispiele für die Verbindungen der Formel (III) seien genannt:

Methyl-, Ethyl-, n-Propyl-, i-Propyl-, n-Butyl-, i-Pentyl-, sec.-Butyl-, tert.-Butyl-, Cyclopropyl-, Cyclopentyl- und Cyclohexylamin; Hydrazin, Methylhydrazin, 1,1-Dimethylhydrazin, Ethylhydrazin und 1,1-Diethylhydrazin.

Das erfindungsgemäße Verfahren (2) zur Herstellung der neuen Verbindungen der Formel (I) wird bevorzugt unter Verwendung von Verdünnungsmitteln durchgeführt. Als Verdünnungsmittel kommen praktisch alle inerten organischen Lösungsmittel in Frage.

Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol, o-Dichlorbenzol, Ether wie Diethyl- und Dibutylether, Glycoldimethylether und Diglycoldimethylether, Tetrahydrofuran und Dioxan und Alkohole wie Methanol, Ethanol, Isopropanol, Glykol.

Das erfindungsgemäße Verfahren (2) wird im allgemeinen bei Temperaturen zwischen −20°C und +50°C durchgeführt. Bevorzugt wird der Bereich zwischen −10°C und 30°C. Die Umsetzungen werden im allgemeinen bei Normaldruck durchgeführt.

Zur Durchführung des erfindungsgemäßen Verfahrens (2) werden die Ausgangsstoffe gewöhnlich annähernd in äquimolaren Mengen eingesetzt. Ein Überschuß der einen oder anderen Reaktionskomponente bringt keine wesentlichen Vorteile. Die Aufarbeitung geschieht nach üblichen Methoden.

Verwendet man beispielsweise für das erfindungsgemäße Verfahren (4) zur Herstellung der erfindungsgemäßen Verbindungen der Formel (II) 4-Chlor-5-fluor-2-methoxy-benzoylessigsäuremethylester und Orthomeisensäuretriethylester als Ausgangsstoffe, so kann die Reaktion durch das folgende Formelschema wiedergegeben werden:

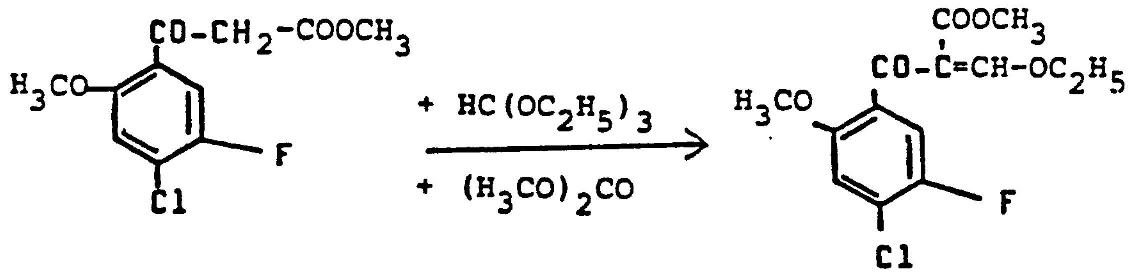


Die als Ausgangsstoffe für das erfindungsgemäße Verfahren (4) zu verwendenden 2-Alkoxy-4-chlor-5-fluor-benzoylessigsäureester sind durch die Formel (IV) allgemin definiert. In dieser Formel steht R bevorzugt für diejenigen Reste, welche oben für Formel (I) angegeben sind.

$R^4$ steht in dieser Formel für Alkyl. Bevorzugt steht $R^4$ für $C_1$—$C_4$-Alkyl, besonders bevorzugt für Methyl, Ethyl, Isopropyl, Butyl.

Die Verbindungen der Formel (IV) sind neu. Sie lassen sich gemäß 6 (oben) herstellen (siehe weiter unten).

Als Beispiele für die Verbindungen der Formel (IV) seien genannt:

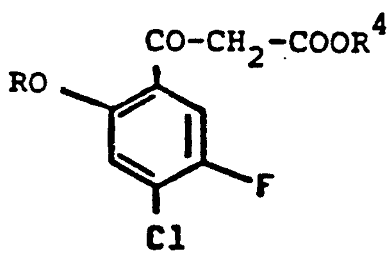

Tabelle 2

| R | $R^4$ | R | $R^4$ | R | $R^4$ |
|---|---|---|---|---|---|
| $CH_3$ | $CH_3$ | $CH_3$ | $C_2H_5$ | $CH_3$ | n-$C_3H_7$ |
| $C_2H_5$ | " | $C_2H_5$ | " | $C_2H_5$ | " |
| n-$C_3H_7$ | " | n-$C_3H_7$ | " | n-$C_3H_7$ | " |
| i-$C_3H_7$ | " | i-$C_3H_7$ | " | i-$C_3H_7$ | " |
| n-$C_4H_9$ | " | n-$C_4H_9$ | " | n-$C_4H_9$ | " |
| i-$C_4H_9$ | " | i-$C_4H_9$ | " | i-$C_4H_9$ | " |
| sec.-$C_4H_9$ | " | sec.-$C_4H_9$ | " | sec.-$C_4H_9$ | " |
| tert.-$C_4H_9$ | " | tert.-$C_4H_9$ | " | tert.-$C_4H_9$ | " |

Die für das erfindungsgemäße Verfahren (4) außerdem als Ausgangsstoffe zu verwendenden Orthoameisensäureester sind durch die Formel (IV) allgemein definiert. $R^3$ steht in dieser Formel für Alkoxy. Bevorzugt steht $R^3$ für $C_1$—$C_4$-Alkoxy.

Die Verbindungen der Formel (V) sind allgemein bekannte Verbindungen der organischen Chemie.

Als Beispiele für die Verbindungen der Formel (V) seien genannt:

Orthoameisensäure-methylester, -ethylester und -propylester.

Das erfindungsgemäße Verfahren (4) zur Herstellung der Verbindungen der Formel (II) wird bevorzugt ohne Verdünnungsmittel durchgeführt.

Das erfindungsgemäße Verfahren (4) wird im allgemeinen bei Temperaturen zwischen 80°C und 180°C durchgeführt. Bevorzugt wird der Bereich zwischen 100°C und 160°C. Die Umsetzungen werden im allgemeinen bei Normaldruck durchgeführt.

Zur Durchführung des erfindungsgemäßen Verfahrens (4) zur Herstellung der Verbindungen der Formel (II) setzt man auf 1 Mol der Verbindung der Formel (IV), 1 bis 2 Mol, vorzugsweise 1,3 bis 1,7 Mol

Orthoameisensäureester der Formel (V) und 2 bis 3 Mol, vorzugsweise 2 bis 2,5 Mol Acetanhydrid ein. Die Aufarbeitung des Reaktionsproduktes geschieht nach üblichen Methoden. Das Reaktionsprodukt kann auch ohne weitere Reinigung für die nächste Umsetzung eingesetzt werden.

Verwendet man beispielsweise für das erfindungsgemäße Verfahren (6) 2-Ethoxy-4-chlor-5-fluor-acetophenon und Kohlensäuredimethylester als Ausgangsstoffe, so kann die Reaktion durch das folgende Formelschema wiedergegeben werden:

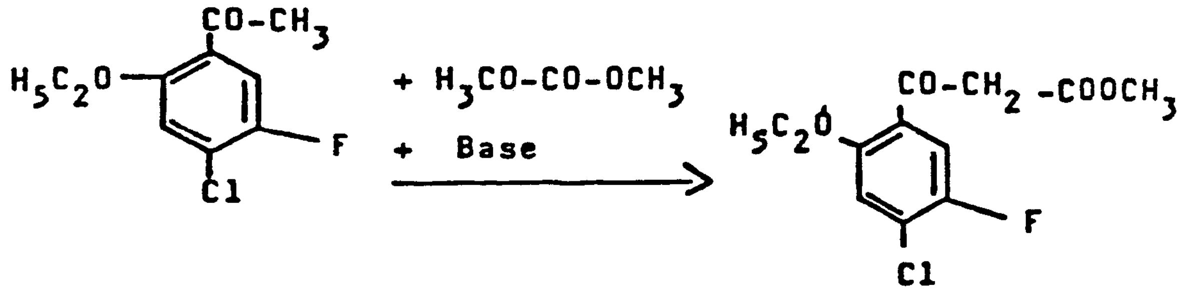


Die als Ausgangsstoffe für das erfindungsgemäße Verfahren (6) zu verwendenden Acetophenone sind durch die Formel (VI) allgemein definiert. In dieser Formel steht R bevorzugt für diejenigen Reste, welche oben für Formel (I) angegeben sind.

Die Verbindungen der Formel (VI) sind neu. Sie lassen sich gemäß 8 (oben) herstellen (siehe weiter unten).

Als Beispiele für die Verbindungen der Formel (VI) seien genannt:

4-Chlor-5-fluor-2-methoxy, 4-Chlor-5-fluor-2-ethoxy, 4-Chlor-5-fluor-2-n-propoxy, 4-Chlor-5-fluor-2-i-propoxy, 4-Chlor-5-fluor-2-n-butoxy, 4-Chlor-5-fluor-2-i-butoxy, 4-Chlor-5-fluor-2-sec.-butoxy- und 4-Chlor-5-fluor-2-tert.-butoxy-acetophenon.

Die für das erfindungsgemäße Verfahren (6) außerdem als Ausgangsstoffe zu verwendenden Kohlensäuredialkylester sind durch die Formel (VII) allgemein definiert. In dieser Formel steht $R^4$ für Alkyl. Bevorzugt steht $R^4$ für $C_1$—$C_4$-Alkyl.

Die Verbindungen der Formel (VII) sind bekannte Verbindungen der organischen Chemie.

Als Beispiele für die Verbindungen der Formel (VII) seien genannt:

Kohlensäuredimethylester, Kohlensäurediethylester und Kohlensäuredi-n-propylester.

Das erfindungsgemäße Verfahren (6) zur Herstellung der Verbindungen der Formel (IV) wird vorzugsweise ohne Verdünnungsmittel durchgeführt. Bevorzugt wird in Gegenwart von überschüssigem Kohlensäuredialkylester der Formel (VII) gearbeitet.

Das erfindungsgemäße Verfahren (6) wird in Gegenwart von starken Basen durchgeführt. Besonders bewährt haben sich Alkalialkoholate, wie Natrium- und Kaliummethylat oder -ethylat und Kalium-tert.-butylat und Alkalihydroxide, wie Natrium- und Kaliumhydroxid.

Das erfindungsgemäße Verfahren (6) wird im allgemeinen bei Temperaturen zwischen 0°C und 140°C durchgeführt. Bevorzugt wird der Bereich zwischen 20°C und 120°C. Die Umsetzungen werden im allgemeinen bei Normaldruck durchgeführt.

Das erfindungsgemäße Verfahren (6) setzt man auf 1 Mol der Verbindung der Formel (IV), 1 bis 3 Mol, vorzugsweise 1 bis 2,0 Mol einer starken Base und 5 bis 30 Mol, vorzugsweise 10 bis 25 Mol Kohlensäuredialkylester der Formel (VII) ein. Die Aufarbeitung erfolgt nach üblichen Methoden.

Verwendet man beispielsweise für das erfindungsgemäße Verfahren (8) 4-Chlor-5-fluor-2-hydroxyacetophenon der Formel (VIII) und Methyliodid bzw. Dimethylsulfat als Ausgangsstoffe, so kann die Reaktion durch das folgende Formelschema wiedergegeben werden:

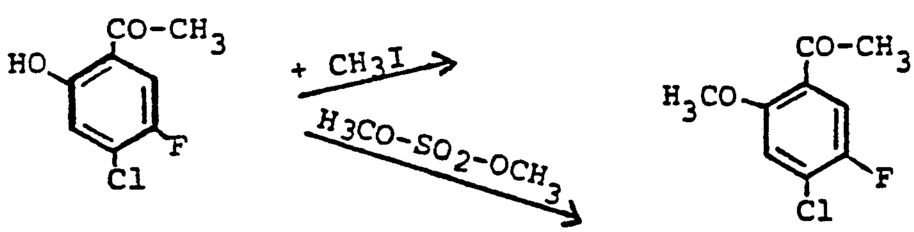


Das als Ausgangsverbindung für das erfindungsgemäße Verfahren (8) zu verwendende 4-Chlor-5-fluor-2-hydroxyacetophenon der Formel (VIII) ist neu. Die Verbindung läßt sich gemäß 10 (oben) herstellen (genaue Beschreibung siehe weiter unten).

Die für das erfindungsgemäße Verfahren (8) außerdem als Ausgangsstoffe zu verwendenden Alkylhalogenide bzw. Dialkylsulfate sind durch die Formel (IX) bzw. (X) allgemein definiert. In dieser Formel steht R bevorzugt für diejenigen Reste welche oben für Formel (I) angegeben sind.

Die Verbindungen der Formeln (IX) und (X) sind allgemein bekannte Verbindungen der organischen Chemie. Als Beispiele für die Alkylhalogenide der Formel (IX) seien genannt:

Methylchlorid, Methylbromid, Methyliodid, Ethylbromid, Ethyliodid, n-Propylbromid, n-Propyliodid, i-Propylbromid, i-Propyliodid, n-Butylbromid, n-Butyliodid, i-Butylbromid, i-Butyliodid, sec.-Butylbromid, sec.-Butyliodid, tert.-Butylbromid und tert.-Butyliodid.

Als Beispiele für die Dialkylsulfate der Formel (X) seien genannt:

Dimethylsulfat, Diethylsulfat und Di-n-propylsulfat.

Das erfindungsgemäße Verfahren (8) zur Herstellung der Verbindungen der (VI) wird vorzugsweise in Gegenwart von Wasser oder polaren organischen Solventien durchgeführt. Bevorzugt in Betracht kommen hierbei Ketone, wie z.B. Aceton, Methylethylketon, Methyl-isopropylketon und Methyl-isobutylketon, ferner Nitrile wie z.B. Acetonitril und Propionitril, außerdem Amide, wie Dimethylformamid, Dimethylacetamid und N-Methylpyrrolidon, sowie Ether, wie Diethyl- und Dibutylether, Glycoldimethylether und Diglycoldimethylether, Tetrahydrofuran und Dioxan.

Als Säureakzeptoren können bei der Durchführung des erfindungsgemäßen Verfahrens (8) vorzugsweise stark basische, jedoch schwach nucleophile Stoffe eingesetzt werden. Bevorzugt infrage kommen Natriumhydrid, Kaliumhydrid, Calciumhydrid, Natriumcarbonat, Kaliumcarbonat sowie Calciumcarbonat und Diazabicyclooctan (DABCO), Diazabicyclononen (DBN) und Diazabicycloundecen (DBU).

Die Reaktionstemperatur kann beim erfindungsgemäßen Verfahren (8) zur Herstellung der neuen Verbindungen der Formel (II) innerhalb eines größeren Bereiches variiert werden. Im allgemeinen arbeitet man bei Temperaturen zwischen 0°C und +100°C, vorzugsweise zwischen 0°C und 80°C. Das erfindungsgemäße Verfahren (8) wird im allgemeinen unter Normaldruck durchgeführt.

Zur Durchführung des erfindungsgemäßen Verfahrens (8) setzt man auf 1 Mol der Verbindung der Formel (VIII), 1 bis 1,5 Mol, vorzugsweise 1 bis 1,2 Mol, Alkylierungsmittel der Formel (IX) bzw. (X) und 1 bis 1,5 Mol, vorzugsweise 1 bis 1,2 Mol, Säureakzeptor ein.

Die Umsetzungen werden im allgemeinen in einem geeigneten Verdünnungsmittel und gegebenenfalls in Gegenwart eines Säureakzeptors durchgeführt. Die Aufarbeitung geschieht nach üblichen Methoden.

Verwendet man beispielsweise für das erfindungsgemäße Verfahren (10) 3-Chlor-4-fluorphenol und Acetylchlorid als Ausgangsstoffe, so kann die Reaktion durch das folgende Formelschema wiedergegeben werden:

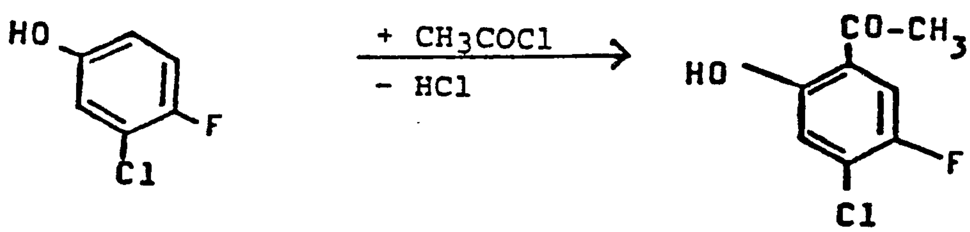


Für das Verfahren (10) wird die Verbindung 3-Chlor-4-fluorphenol der Formel (XI) als Ausgangsverbindung verwendet.

3-Chlor-4-fluorphenol ist eine allgemein bekannte Verbindung der organischen Chemie.

Die für das Verfahren (10) außerdem als Ausgangsstoffe zu verwendenden Acetylierungsmittel sind durch die Formel (XII) allgemein definiert. In dieser Formel steht W für Halogen, wie insbesondere Fluor, Chlor oder Brom oder für den Rest $CH_3COO$—.

Die Verbindungen der Formel (XII) sind allgemein bekannte Verbindungen der organischen Chemie.

Als Beispiele für die Verbindungen der Formel (XII) seien genannt:

Acetyl-fluorid, -chlorid und -bromid und Essigsäureanhydrid.

Die Acylierung wird in Gegenwart bekannter Acylierungskatalysatoren durchgeführt. Als Acylierungskatalysatoren kommen für das Verfahren (10) bevorzugt Friedel-Crafts-Katalysatoren infrage. Hierzu gehören Aluminiumhalogenide, wie Aluminiumtrichlorid sowie Bortrifluorid.

Das erfindungsgemäße Verfahren (10) wird bevorzugt ohne Verdünnungsmittel durchgeführt.

Das erfindungsgemäße Verfahren (10) wird im algemeinen bei Temperaturen zwischen 0°C und 120°C durchgeführt. Bevorzugt wird der Bereich zwischen 20°C und 100°C. Die Umsetzungen werden im allgemeinen bei Normaldruck durchgeführt.

Zur Durchführung des erfindungsgemäßen Verfahrens (10) setzt man auf 1 Mol 3-Chlor-4-fluorphenol der Formel (XI), 1 bis 2,2 Mol, vorzugsweise 1 bis 1,8 Mol, Acetylierungsmittel der Formel (XII) und 1 bis 3,5 Mol, vorzugsweise 1 bis 2,8 Mol, Acylierungskatalysator ein.

Die Aufarbeitung erfolgt in üblicher Weise z.B. durch Zugabe von Eis oder Eiswasser zum Reaktionsgemisch nach beendeter Reaktion und durch Absaugen des erkaltenen Produkts.

Die Verbindungen der Formel (I) können als Zwischenprodukte für die Synthese von Oxochinolincarbonsäure-Derivaten verwendet werden, die eine hervorragende bakterizide Wirksamkeit besitzen (vgl. z.B. EP—OS 78 362). Diese lassen sich daraus gemäß folgendem Formelschema herstellen:

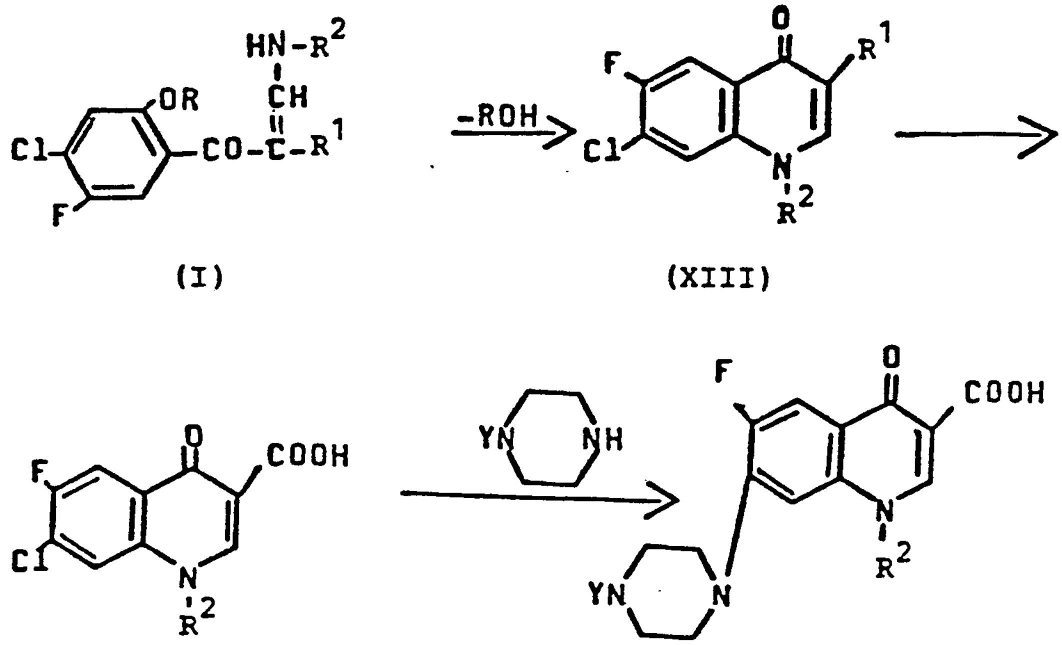


(I) (XIII)

(XIV) (XV)

Y = H, Alkyl

Die Cyclisierung der Verbindungen der Formel (I) zu der bekannten Verbindungen der Formel (XIII) wird in Gegenwart von Verdünnungsmitteln und in Gegenwart von Säureakzeptoren bei Temperaturen zwischen 60°C bis 300°C vorzugsweise bei 80°C bis 180°C, durchgeführt.

Als Verdünnungsmittel können Dioxan, Dimethylsulfoxid, N-Methyl-pyrrolidon, Sulfolan, Hexamethylphosphorsäuretriamid und bevorzugt, N,N-Dimethylformamid verwendet werden.

Als Säureakzeptoren kommen für diese Umsetzung Kalium-tert.-butanolat, Butyl-lithium, Lithiumphenyl, Phenyl-magnesiumbromid, Natriummethylat, Natriumhydrid und besonders bevorzugt Kalium- oder Natriumcarbonat in Betracht.

Bei der Durchführung der Cyclisierung setzt man auf 1 Mol der Verbindung der Formel (I) 1 bis 1,6 Mol, vorzugsweise 1 bis 1,3 Mol Säureakzeptor ein.

Die Aufarbeitung des Reaktionsproduktes erfolgt in üblicher Weise. Die weiteren Umsetzungen der Verbindungen der Formel (XIII) zu den bekannten Verbindungen der Formeln (XIV) und (XV) sind bereits beschrieben worden (vgl. z.B. EP—OS 78 362 und EP—OS 113 092).

## Herstellungsbeispiele

### Beispiel 1

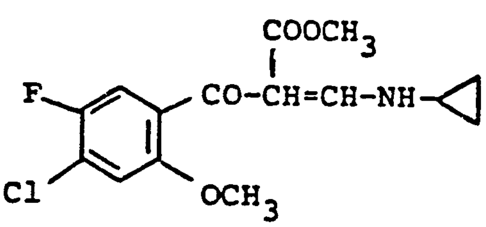

Eine Lösung von 10,3 g (0,034 Mol) 2-(4-Chlor-5-fluor-2-methoxy-benzoyl)-3-methoxy-acrylsäuremethylester in 40 ml Ethanol wird unter Eiskühlung und Rühren tropfenweise mit 2 g (0,034 Mol) Cyclopropylamin versetzt und 2 Studen bei 20°C weitergerührt. Der Niederschlag wird kalt abgesaugt und mit wenig Ethanol nachgewaschen.

Man erhält 5,5 g (48,6% der Theorie) 2-(4-Chlor-5-fluor-2-methoxy-benzoyl)-3-cyclopropylamino-acrylsäuremethylester vom Schmelzpunkt 128°C—132°C.

Herstellung der Verbindungen der Formel (II)

Beispiel (II-1)

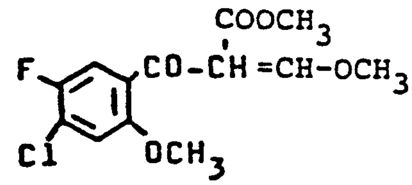

Ein Gemisch aus 9,6 g (0,037 Mol) 4-Chlor-5-fluor-2-methoxy-benzoylessigsäuremethylester, 5,5 g (0,054 Mol) Orthoameisensäuretrimethylester und 8,7 g (0,087 Mol) Essigsäureanhydrid wird 3 Stunden unter Rückfluß erhitzt. Anschließend werden bei Normaldruck im Wasserstrahlvakuum und zuletzt im Hochvakuum bei einer Badtemperatur von 120°C bis 130°C die flüchtigen Bestandteile abdestilliert.

Man erhält 10,3 g rohrer 2-(4-Chlor-5-fluor-2-methoxybenzoyl)-3-methoxy-acrylsäuremethylester. Das Produkt kann ohne weitere Aufarbeitung in die nächste Reaktionsstufe eingesetzt werden.

Herstellung der Verbindungen der Formel (IV)

Beispiel (IV-1)

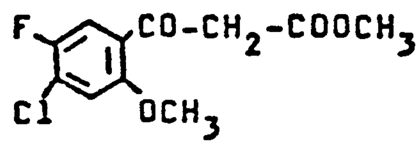

Aus einer Mischung von 210 ml Kohlensäuredimethylester une 8,9 g (0,165 Mol) Natriummethylat destilliert man 30 ml Kohlensäuredimethylester ab und tropft dann unter Sieden innerhalb von 3 Stunden eine Lösung von 30,4 g (0,15 Mol) 4-Chlor-5-fluor-2-methoxy-acetophenon in 75 ml Kohlensäuremethylester zum Reaktionsgemisch. Das bei der Reaktion entstehende Methanol wird dabei über eine Kolonne abdestilliert. Nach der Zugabe wird noch 3 Stunden unter Rückfluß gekocht, dann destilliert man das Lösungsmittel im Vakuum ab. Den Rückstand löst man in 500 ml Wasser, gibt bei 10°C konzentrierte Salzsäure zu bis ein pH von 6 erreicht ist und extrahiert dann zweimal mit je 200 ml Methylenchlorid. Die organische Phase wird über Natriumsulfat getrocknet, im Vakuum eingedampft und dann im Hochvakuum bei 120°C an destilliert. Auf diese Weise erhält man 2,4 g des eingesetzten 4-Chlor-5-fluor-2-methoxy-acetophenons zurück.

Als Rückstand bleiben 32 g (89% der Theorie) 4-Chlor-5-fluor-2-methoxy-benzoylessigsäuremethylester in Form eines hellbraunen Pulvers mit den Schmelzpunkt 61°C zurück.

Herstellung der Verbindungen der Formel (VI)

Beispiel (VI-1)

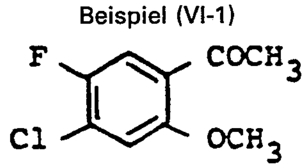

Ein Gemisch aus 7,5 g (0,04 Mol) 4-Chlor-5-fluor-2-hydroxy-acetophenon, 8,3 g (0,06 Mol) Kaliumcarbonat, 50 ml Acetonitril und 6,3 g (0,044 Mol) Methyliodid wird 7 Studen bei 55°C gerührt. Anschließend wird das Reaktionsgemisch filtriert und das Filtrat im Vakuum eingedampft. Der Rückstand wird mit 50 ml Wasser verrieben und abgesaugt.

Man erhält so 7,4 g (92% der Theorie) 4-Chlor-5-fluor-2-methoxy-acetophenon in Form eines beigen Pulvers mit dem Schmelzpunkt 80°C.

Herstellung der Verbindung der Formel (VIII)

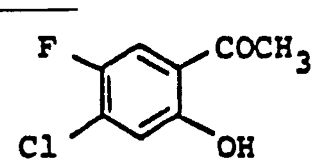

Zu einer Mischung aus 11,8 g (0,08 Mol) 3-Chlor-4-fluor-phenol und 26,8 g (0,2 Mol) Aluminiumchlorid tropft man bei 20 bis 30°C 9,5 g (0,12 Mol) Acetylchlorid. Das Gemisch wird 1 Stunde bei 95 bis 100°C

gerührt und dann noch heiß auf ein Gemisch aus 300 g Eis und 100 ml Wasser gegeben. Nach 30 Minuten saugt man das augefallene Produkt ab und wäscht gut mit Wasser nach.

Man erhält so 12,9 g (86% der Theorie) 4-Chlor-5-fluor-2-hydroxy-acetophenon in Form eines beigen Pulvers mit dem Schmelzpunkt 72°C.

Herstellung der Verbindungen der Formel (XIII)

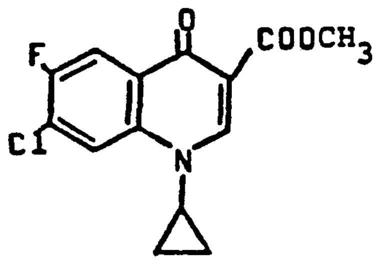

Eine Suspension von 5,2 g (0,016 Mol) 2-(4-Chlor-5-fluor-2-methoxy-benzoyl)-3-cyclopropylamino-acrylsäuremethylester (Beispiel 1), 2,6 g (0,019 Mol) Kaliumcarbonat und 25 ml Dimethylformamid wird 2 Stunden auf ca. 150°C erhitzt. Anschließend wird das Reaktionsgemisch auf Eis gegossen, der Niederschlag abgesaugt und mit Wasser nachgewaschen. Nach dem Trocknen im Vakuumtrockenschrank bei 100°C werden 3,6 g Rohprodukt erhalten.

Nach Umkristallisation aus Ethanol erhält man 3,1 g (66% der Theorie) 7-Chlor-1-cyclopropyl-6-fluor-1,4-dihydro-4-oxo-chinolincarbonsäuremethylester vom Schmelzpunkt 244°C—245°C.

**Patentansprüche**

1. Aminoacrylsäure-Derivate der Formel (I)

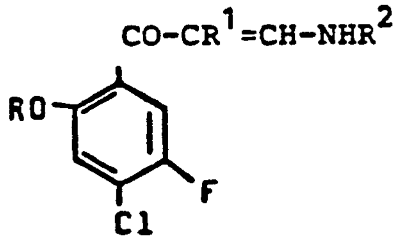

(I)

in welcher
R für Alkyl steht,
$R^1$ für Alkoxycarbonyl steht und
$R^2$ für Alkyl, Cycloalkyl, Amino, Alkylamino oder Di-alkylamino steht.

2. Verfahren zur Herstellung der neuen Aminoacrylsäure-Derivate der Formel (I)

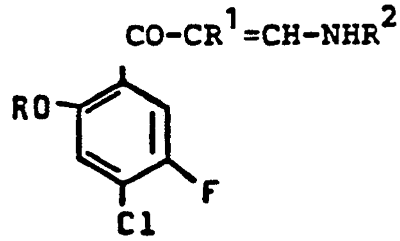

(I)

in welcher
R für Alkyl steht,
$R^1$ für Alkoxycarbonyl steht und
$R^2$ für Alkyl, Cycloalkyl, Amino, Alkylamino oder Di-alkylamino steht,
dadurch gekennzeichnet, daß man Acrylsäure-Derivate der Formel (II)

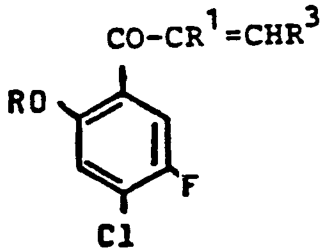

(II)

in welcher

R und $R^1$ die oben angegebenen Bedeutungen haben und

$R^3$ für Alkoxy steht

mit Aminen der Formel (III)

$$R^1NH_2 \qquad (III)$$

in welcher

$R^2$ die oben angegebene Bedeutung hat,

gegebenenfalls in Gegenwart von inerten Verdünnungsmittel umsetzt.

3. Acrylsäure-Derivate der Formel (II)

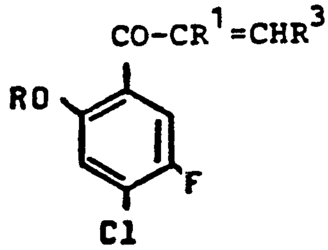

(II)

in welcher

R für Alkyl steht,

$R^1$ für Alkoxycarbonyl steht,

$R^3$ für Alkoxy steht.

4. Verfahren zur Herstellung der Acrylsäure-Derivate der Formel (II),

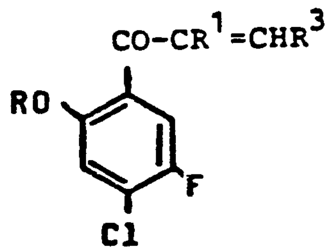

(II)

in welcher

R für Alkyl steht,

$R^1$ für Alkoxycarbonyl steht,

$R^3$ für Alkoxy steht,

dadurch gekennzeichnet daß man 2-Alkoxy-4-chlor-5-fluor-benzoylessigsäureester der Formel (IV)

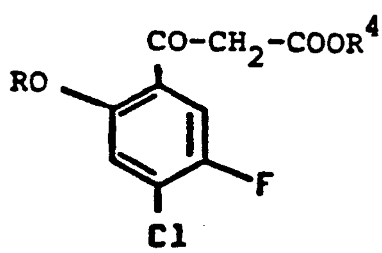

(IV)

in welcher

R die oben angegebene Bedeutung hat und

$R^4$ für Alkyl steht,

mit Orthoameisensäureestern der Formel (V)

$$HC(R^3)_3 \qquad (V)$$

in welcher

$R^3$ die oben angegebene Bedeutung hat,

in Gegenwart von Acetanhydrid und gegebennfalls in Gegenwart von Verdünnungsmitteln umsetzt.

5. 2-Alkoxy-4-chlor-5-fluor-benzoylesseigsäureester der Formel (IV)

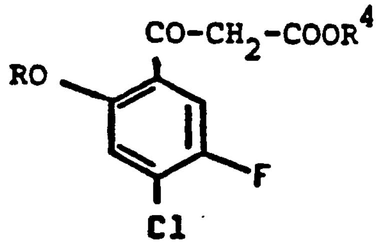

(IV)

in welcher
R für Alkyl steht und
$R^4$ für Alkyl steht.

6. Verfahren zur Herstellung der neuen 2-Alkoxy-4-chlor-5-fluor-benzoylessigsäureester der Formel (IV),

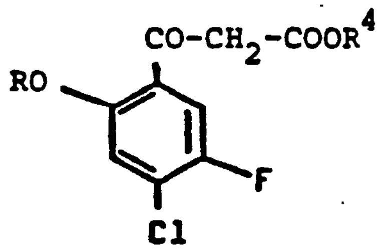

(IV)

in welcher
R für Alkyl steht und
$R^4$ für Alkyl steht,
dadurch gekennzeichnet, daß man Acetophenone der Formel (VI)

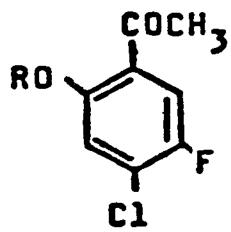

(VI)

in welcher
R die oben angegebene Bedeutung hat,
mit Kohlensäuredialkylestern der Formel (VII)

$$R^4O—CO—OR^4 \quad (VII)$$

in welcher
$R^4$ die (oben) angegebene Bedeutung hat,
in Gegenwart von starken Basen umsatzt.

7. Acetophenone der Formel (VI)

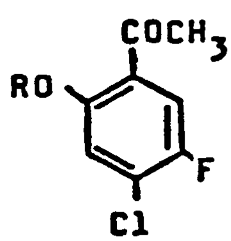

(VI)

in welcher
R für Alkyl steht.

8. Verfahren zur Herstellung der Acetophenone der Formel (VI)

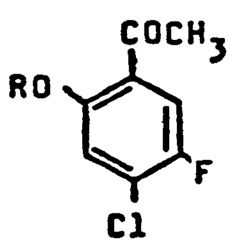

(VI)

in welcher
R für Alkyl steht,
dadurch gekennzeichnet, daß man 4-Chlor-5-fluor-2-hydroxyacetophenon der Formel (VIII)

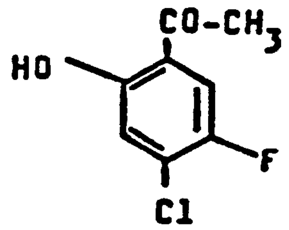

(VIII)

α) mit Alkylhalogeniden der Formel (IX)

R—Hal (IX)

in welcher
R die oben angegebene Bedeutung hat und Hal für Halogen steht, oder
β) mit Dialkylsulfaten der Formel (X)

$RO—SO_2—OR$ (X)

in welcher
R die oben angegebene Bedeutung hat,
in Gegenwart von Säureakzeptoren und in Gegenwart von Verdünnungsmitteln umsetzt.

9. 4-Chlor-5-fluor-2-hydroxyacetophenon der Formel (VIII)

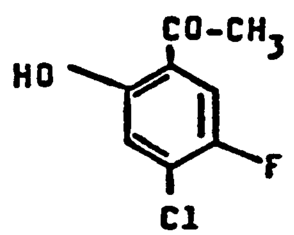

(VIII)

10. Verfahren zur Herstellung von 4-Chlor-5-fluor-2-hydroxyacetophenon der Formel (VIII), dadurch gekennzeichnet, daß man 3-Chlor-4-fluorphenol der Formel (XI)

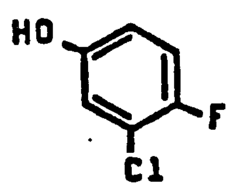

(XI)

mit Acetylierungsmitteln der Formel (XII)

$$CH_3\overset{\overset{O}{\|}}{C}—W$$ (XII)

in welcher
W für Halogen oder den Rest $CH_3$—COO— steht,
in Gegenwart von Acylierungskatalysatoren und gegebenenfalls in Gegenwart von Verdünnungsmitteln umsetzt.

11. Verwendung von Verbindungen der Formel (I) gemäß 1 (oben), zur Herstellung von Oxochinolincarbonsäure-Derivaten, dadurch gekennzeichnet, daß man die Verbindungen der Formel (I) cyclisiert und anschließend nach bekannten Methoden zu Oxochinolincarbonsäure-Derivaten umsetzt.

**Revendications**

1. Dérivés de l'acide aminoacrylique de la formule (I)

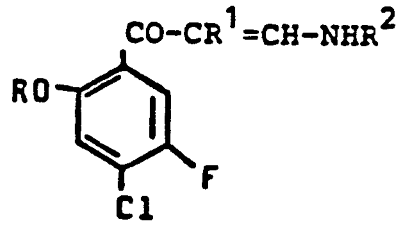

(I)

dans laquelle
R représente un radical alkyle
$R^1$ représente un radical alcoxycarbonyle et
$R^2$ représente un radical alkyle, cycloalkyle, amino, alkylamino ou dialkylamino.

2. Procédé de production des nouveaux dérivés de l'acide aminoacrylique de la formule (I)

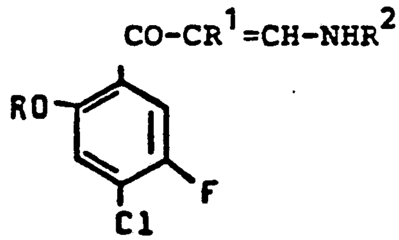

(I)

dans laquelle
R représente un radical alkyle
$R^1$ représente un radical alcoxycarbonyle et
$R^2$ représente un radical alkyle, cycloalkyle, amino, alkylamino ou dialkylamino,
caractérisé en ce que l'on réagir des dérivés de l'acide acrylique de la formule (II)

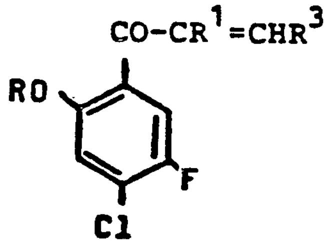

(II)

dans laquelle
R et $R^1$ ont la signification mentionnée ci-dessus et
$R^3$ représente un radical alcoxy
avec des amines de la formule (III)

$R^1NH_2$ (III)

dans laquelle
$R^2$ a la signification mentionnée ci-dessus,
éventuellement en présence de diluants inertes.

3. Dérivés de l'acide acrylique de la formule (II)

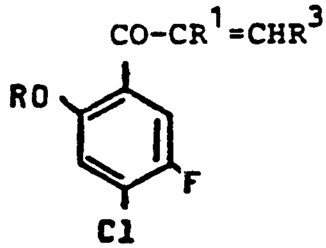

(II)

dans laquelle
R représente un radical alkyle

$R^1$ représente un radical alcoxycarbonyle
$R^3$ représente un radical alcoxy.

4. Procédé de production des dérivés de l'acide acrylique de la formule (II)

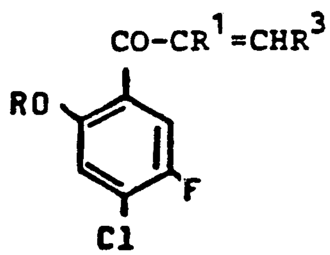

(II)

dans laquelle
R représente un radical alkyle
$R^1$ représente un radical alcoxycarbonyle
$R^3$ représente un radical alcoxy
caractérisé en ce que l'on fait réagir des esters de l'acide 2-alcoxy-4-chloro-5-fluoro-benzoyl-acétique de la formule (IV)

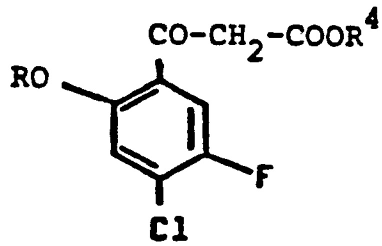

(IV)

dans laquelle
R a la signification mentionée ci-dessus et
$R^4$ représente un radical alkyle
avec des esters de l'acide orthoformique de la formule (V)

$$HC(R^3)_3 \quad (V)$$

dans laquelle
$R^3$ a la signification mentionnée ci-dessus,
en présence d'anhydride acétique et éventuellement en présence de diluants.

5. Esters de l'acide 2-alcoxy-4-chloro-5-fluoro-benzoyl-acétique de la formule (IV)

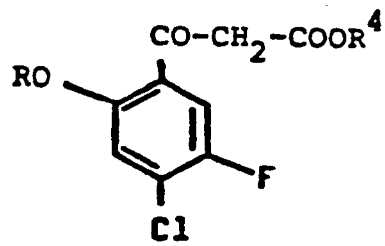

(IV)

dans laquelle
R représente un radical alkyle et
$R^4$ représente un radical alkyle.

6. Procédé de production des nouveaux esters de l'acide 2-alcoxy-4-chloro-5-fluoro-benzoyl-acétique de la formule (IV)

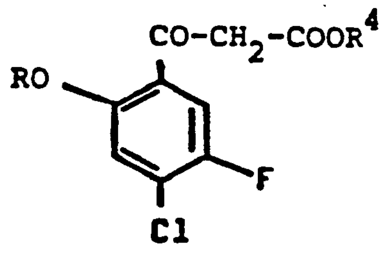

(IV)

dans laquelle
R représente un radical alkyle et

$R^4$ représente un radical alkyle,

caractérisé en ce que l'on fait réagir des acétophénones de la formule (VI)

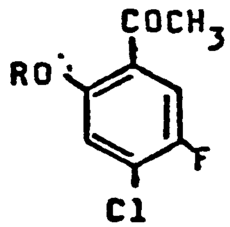

(VI)

dans laquelle

R a la signification mentionnée ci-dessus,

avec des ester dialkyliques de l'acide carbonique de la formule (VII)

$$R^4O—CO—OR^4 \qquad (VII)$$

dans laquelle

$R^4$ a la signification mentionnée ci-dessus,

en présence de bases fortes.

7. Acétophénones de la formule (VI)

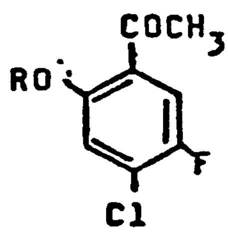

(VI)

dans laquelle

R représente un radical alkyle.

8. Procédé de production des acétophénones de la formule (VI)

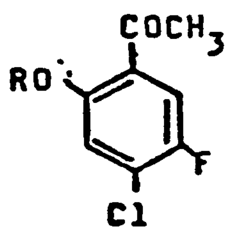

(VI)

dans laquelle

R représente un radical alkyle,

caractérisé en ce que l'on fait réagir la 4-chloro-5-fluoro-2-hydroxy-acétophénone de la formule (VIII)

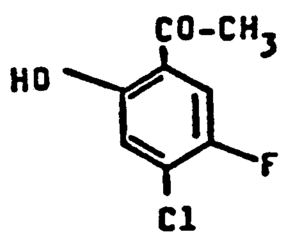

(VIII)

α) avec des halogénures d'alkyle de la formule (IX)

$$R—Hal \qquad (IX)$$

dans laquelle

R a la signification mentionnée ci-dessus et

Hal représente un halogène ou

β) avec des sulfates dialkyliques de la formule (X)

$$RO—SO_2—OR \qquad (X)$$

dans laquelle
R a la signification mentionnée ci-dessus,
en présence d'accepteurs d'acides et en présence de diluants.

9. 4-chloro-5-fluoro-2-hydroxyacétophénone de la formule (VIII)

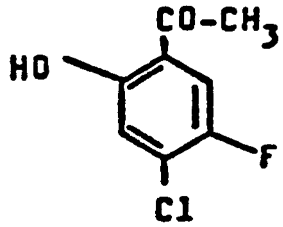

(VIII)

10. Procédé de production de 4-chloro-5-fluoro-2-hydroxy-acétophénone de la formule (VIII), caractérisé en ce que l'on fait réagir du 3-chloro-4-fluorophénol de la formule (XI)

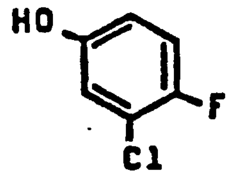

(XI)

avec des agents d'acétylation de la formule (XII)

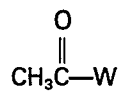

(XII)

dans laquelle
W représente un halogène ou le radical $CH_3$—COO,
en présence de catalyseurs d'acylation et éventuellement en présence de diluants.

11. Utilisation des composés de la formule (I) suivant (1) ci-dessus pour la production de dérivés de l'acide oxoquinoléine-carboxylique, caractérisée en ce que l'on effectue une cyclisation des composés de la formule (I) et qu'on les transforme ensuite en dérivés de l'acide oxoquinoléine-carboxylique par des procédés connus.

**Claims**

1. Aminoacrylic acid derivatives of the formula (I)

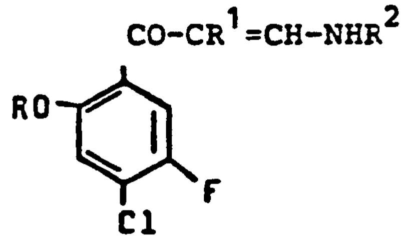

(I)

in which
R represents alkyl,
$R^1$ represents alkoxycarbonyl and
$R^2$ represents alkyl, cycloalkyl, amino, alkylamino or dialkylamino.

2. Process for the preparation of the new aminoacrylic acid derivatives of the formula (I)

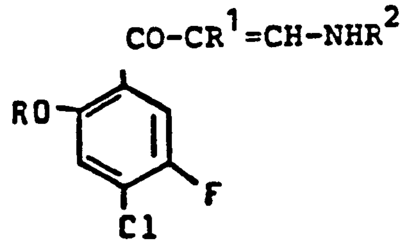

(I)

in which

R represents alkyl,

$R^1$ represents alkoxycarbonyl and

$R^2$ represents alkyl, cycloalkyl, amino, alkylamino or dialkylamino,

characterized in that acrylic acid derivatives of the formula (II)

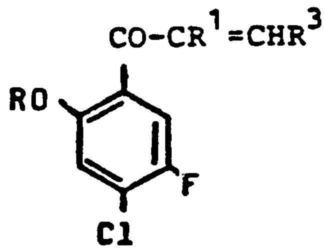

(II)

in which

R and $R^1$ have the meanings given above and

$R^3$ represents alkoxy,

are reacted with amines of the formula (III)

$$R^1NH_2 \qquad (III)$$

in which

$R^2$ has the meaning given above,

if appropriate in the presence of inert diluents.

3. Acrylic acid derivatives of the formula (II)

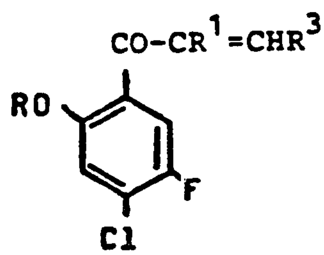

(II)

in which

R represents alkyl,

$R^1$ represents alkoxycarbonyl and

$R^3$ represents alkoxy.

4. Process for the preparation of the acrylic acid derivatives of the formula (II)

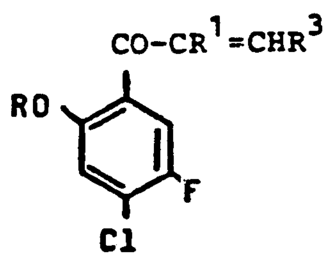

(II)

in which

R represents alkyl,

$R^1$ represents alkoxycarbonyl,

$R^3$ represents alkoxy,

characterized in that 2-alkoxy-4-chloro-5-fluoro-benzoylacetates of the formula (IV)

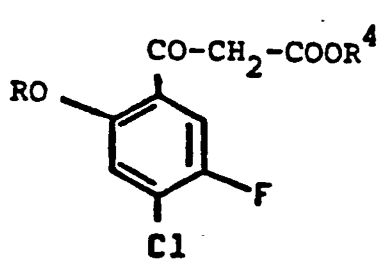

(IV)

in which
R has the meaning given above and
$R^4$ represents alkyl,
are reacted with orthoformates of the formula (V)

$$HC(R^3)_3 \qquad (V)$$

in which
$R^3$ has the meaning given above,
in the presence of acetic anhydride and, if appropriate in the presence of diluents.

5. 2-Alkoxy-4-chloro-5-fluoro-benzoylacetates of the formula (IV)

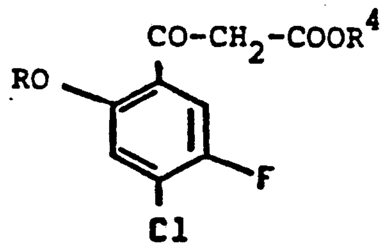

(IV)

in which
R represents alkyl and
$R^4$ represents alkyl.

6. Process for the preparation of the new 2-alkoxy-4-chloro-5-fluoro-benzoylacetates of the formula (IV)

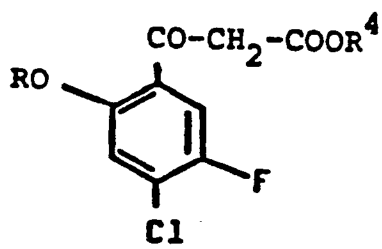

(IV)

in which
R represents alkyl and
$R^4$ represents alkyl,
characterized in that acetophenones of the formula (VI)

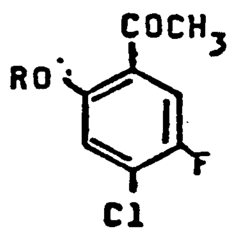

(VI)

in which
R has the meaning given above,
are reacted with dialkyl carbonates of the formula (VII)

$$R^4O—CO—OR^4 \qquad (VII)$$

in which
$R^4$ has the meaning given (above),
in the presence of strong bases.

7. Acetophenones of the formula (VI)

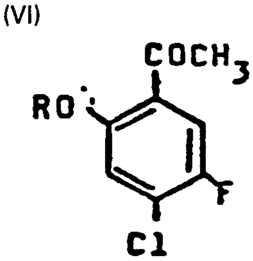

(VI)

in which

R represents alkyl.

8. Process for the preparation of the acetophenones of the formula (VI)

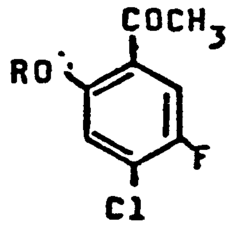

(VI)

in which

R represents alkyl,

characterized in that 4-chloro-5-fluoro-2-hydroxyacetophenone of the formula (VIII)

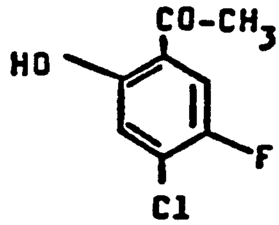

(VIII)

is reacted

α) with alkyl halides of the formula (IX)

R—Hal (IX)

in which

R has the meaning given above and

Hal represents halogen, or

β) with dialkyl sulphates of the formula (X)

$RO—SO_2—OR$ (X)

in which

R has the meaning given above,

in the presence of acid acceptors and in the presence of diluents.

9. 4-Chloro-5-fluoro-2-hydroxyacetophenone of the formula (VIII)

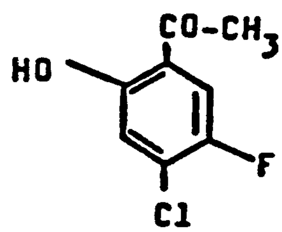

(VIII)

10. Process for the preparation of 4-chloro-5-fluoro-2-hydroxyacetophenone of the formula (VIII), characterized in that 3-chloro-4-fluorophenol of the formula (XI)

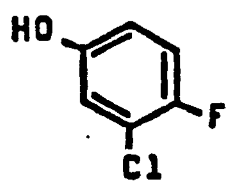

(XI)

is reacted with actylating agents of the formula (XII)

$$CH_3\overset{\overset{O}{\|}}{C}—W$$ (XII)

in which

W represents halogen or the radical $CH_3$—COO—, in the presence of acylation catalysts and, if appropriate, in the presence of diluents.

11. Use of compounds of the formula (I) according to 1 (above) for the preparation of oxoquinolinecarboxylic acid derivatives, characterized in that the compounds of the formula (I) are cyclised and then converted by known methods into oxoquinolinecarboxylic acid derivatives.